# FASCICULE DE BREVET EUROPEEN

(11) **EP 1 584 330 B1**
(45) Date de publication et mention de la délivrance du brevet: **04.07.2007**
(21) Numéro de dépôt: 05290591.6
(22) Date de dépôt: 17.03.2005
(51) Int. Cl.: A61K 8/04, A61K 8/92, A61K 8/26, A61K 8/28, A61Q 15/00

(54) **Composition anti-transpirante du type émulsion huile dans eau contenant des microparticules de cire**
Wachsmikropartikel enthaltende schweisshemmende Zusammensetzung in Form einer Öl-in-Wasser Emulsion
Oil in water emulsion-type antiperspirant composition containing wax microparticles

(30) Priorité: 07.04.2004 FR 0450698
(43) Date de publication de la demande: 12.10.2005
(73) Titulaire: L'ORÉAL, 75008 Paris (FR)
(72) Inventeur: Aubert, Lionnel, 95270 Asnieres sur Oise (FR); Douin, Véronique, 78860 Saint-Nom la Bretèche (FR); Provost, Roxane, 75013 Paris (FR)
(74) Mandataire: Miszputen, Laurent

(56) Documents cités:
- EP-A- 0 853 940
- EP-A- 1 541 123
- WO-A-01/85119
- WO-A-03/105795
- WO-A-20/04022010
- DE-A- 10 021 167
- US-B1- 6 261 543

## Description

L'invention concerne une composition cosmétique comprenant dans un support du type émulsion huile dans eau :
(A) au moins un actif anti-transpirant ;
(B) au moins des particules de cire ayant une taille moyenne inférieure à 50µm et un point de fusion supérieur à 80 °C.

La présente invention a pour objet également un procédé cosmétique de traitement des odeurs corporelles liées à la transpiration (notamment odeurs axillaires, odeurs des pieds), consistant à appliquer sur la surface de la peau une quantité efficace d'une telle composition.

Dans le domaine cosmétique, il est bien connu d'utiliser en application topique, des produits déodorants contenant des substances actives de type anti-transpirant, de type bactéricide ou du type absorbeur d'odeurs pour diminuer, voire supprimer, les odeurs corporelles généralement désagréables.

Les substances anti-transpirantes ont pour effet de limiter le flux sudoral. Elles sont généralement constituées de sels d'aluminium.

Bien des types différents de compositions anti-transpirantes ont été décrits dans la littérature et sont apparus sur le marché sous des formes telles que des gels, des sticks, des crèmes, des roll-ons ou des aérosols.

Parmi les roll-ons ou les crèmes disponibles sur le marché des produits anti-transpirants, il existe trois grands types de formulations : les formulations anhydres ou soft solid, les émulsions eau-dans-huile et les émulsions huile-dans-eau .

Les formulations anhydres soft solid et les émulsions eau-dans-huile qui sont des supports a phase huileuse continue présentent l'inconvénient de produire un toucher gras à l'application et un manque de sensation de fraîcheur voire une efficacité déodorante insuffisante.

Les émulsions huile-dans-eau anti-transpirantes à phase aqueuse continue sont particulièrement recherchées pour leurs qualités cosmétiques originales au niveau de leur pénétration, de leur étalement et de la sensation de fraîcheur sur la peau après application. Les compositions actuellement sur le marché présentent comme inconvénients
a) un effet mouillé à l'application pouvant entraîner un transfert du produit au contact des vêtements et qui est dû à un séchage trop lent ;
b) un effet collant dû à la présence de sels anti-transpirants qui sont à l'état dissous dans la phase aqueuse.

Ainsi, on recherche des émulsions huile-dans-eau anti-transpirantes dont le temps de séchage est plus court et dont l'effet collant dû à la présence des sels anti-transpirants dissous dans la phase aqueuse est sensiblement diminué voire supprimé.

L'utilisation de cires de polyéthylène micronisées comme agent de suspension des sels d'aluminium dans les formulations anti-transpirantes du type roll-on est connue dans l'article de Harvey M. Fishmann dans la revue Happi août 1998 page 40. Les cires de polyéthylène micronisées du type ®MICROTHENE ou ACUMIST sont connues également dans les demandes WO01/85119 et WO03/105795 comme charges dans des formulations anti-transpirantes sous forme d'émulsion eau-dans-huile. Dans les documents US 5,294,447, US 5,178,881 et EP981324 les cires de polyéthylène du type ®MICROTHENE sont utilisées comme agent de séchage dans des formulations anti-transpirantes anhydres dans lesquelles l'actif anti-transpirant (sel d'aluminium) est en suspension dans la phase huileuse continue.

La demanderesse a découvert de manière inattendue qu'en ajoutant dans des émulsions huile-dans-eau à base d'actif anti-transpirant des particules de cire ayant une taille moyenne de particule inférieure à 50µm et un point de fusion supérieur à 80°C, on obtenait des temps de séchage rapides, un effet collant fortement réduit ainsi qu'une bonne efficacité déodorante par rapport aux émulsions huile/eau de l'art antérieur.

Cette découverte est à la base de la présente invention.

La présente invention a ainsi pour premier objet une composition cosmétique comprenant dans un support du type émulsion huile dans eau :
(A) au moins un actif anti-transpirant ;
(B) au moins des particules de cire ayant une taille moyenne inférieure à 50µm et un point de fusion supérieur à 80 °C.

La présente invention a pour second objet l'utilisation cosmétique d'une composition telle que définie ci-dessus, pour réduire le flux sudoral et/ou masquer, améliorer ou réduire l'odeur désagréable résultant de la décomposition de la sueur humaine par des bactéries.

La présente invention a pour objet également un procédé cosmétique de traitement de la transpiration, consistant à appliquer sur la surface de la peau une quantité efficace d'une telle composition.

Le traitement de la transpiration peut consister aussi bien à la réduction ou la suppression de l'odeur désagréable provoquée par la sueur que la réduction ou la suppression de la sensation d'humidité produite par la sueur.

La présente invention a pour objet également un procédé cosmétique de traitement des odeurs corporelles liées à la transpiration (notamment odeurs axillaires, odeurs des pieds), consistant à appliquer sur la surface de la peau une quantité efficace d'une telle composition.

La présente invention a pour objet également l'utilisation des particules de cire ayant une taille moyenne inférieure à 50µm et un point de fusion supérieur à 80 °C dans une composition anti-transpirante du type émulsion huile-dans-eau dans le but de réduire le temps de séchage et l'effet collant après application sur la peau.

Au sens de la présente invention, on entend par "actif anti-transpirant" toute substance capable de réduire le flux sudoral.

Les actifs anti-transpirants utilisables selon l'invention sont choisis de préférence parmi les sels d'aluminium et/ou de zirconium ; les complexes d'hydroxychlorure de zirconium et d'hydroxychlorure d'aluminium avec un acide aminé

Parmi les sels d'aluminium, on peut citer en particulier le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

Parmi les sels d'aluminium et de zirconium, on peut citer en particulier l'aluminium zirconium octachlorohydrate, l'aluminium zirconium pentachlorohydrate, l'aluminium zirconium tetrachlorohydrate, l'aluminium zirconium trichlorohydrate.

Les complexes d'hydroxychlorure de zirconium et d'hydroxychlorure d'aluminium avec un acide aminé sont généralement connus sous l'appellation ZAG (lorsque l'acide aminé est la glycine). Ils sont notamment décrits dans le brevet US-3792068. Les complexes ZAG présentent d'ordinaire un quotient Al/Zr allant d'environ 1,67 à 12,5 et un quotient Métal/Cl allant d'environ 0,73 à 1,93. Parmi ces produits on peut citer les complexes aluminium zirconium octachlorohydrex glycine, aluminium zirconium pentachlorohydrex glycine, aluminium zirconium tetrachlorohydrex glycine et aluminium zirconium trichlorohydrex glycine.

On utilisera plus particulièrement le chlorhydrate d'aluminium sous forme activée ou non activée notamment le produit commercialisé par la société REHEIS sous la dénomination ®MICRODRY ALUMINUM CHLOROHYDRATE ou par la société GUILINI CHEMIE sous la dénomination ®ALOXICOLL PF 40. On pourra utiliser également le produit commercial ®REACH 103 fabriqué par la société REHEIS ou le produit commercial WESTCHLOR 200 fabriqué par la société WESTWOOD.

Les actifs anti-transpirants sont de préférence présents dans la composition selon l'invention à raison d'environ 0,001 à 40% en poids par rapport à la composition totale, et de préférence à raison d'environ 0,1 à 25% en poids.

Les particules de cire micronisées conformes à l'invention ont de préférence une taille moyenne de particules inférieure à 20 µm et plus préférentiellement de 1 à 15 µm. Elles présentent un point de fusion de préférence supérieur à 100°C. Leur densité à 25°C varie en général de 0,9 à 2,5 g/cm³.

Les particules de cire micronisées conformes à l'invention peuvent être constituées de cire choisie parmi les cires naturelles d'origine minérale, fossile, animale ou végétale ; les cires synthétiques ou de leurs mélanges.

Parmi les cires naturelles minérales, on peut citer les cires microcristallines, la paraffine, le pétrolatum, les paraffines, l'ozokérite, la cire de Montan.

Parmi les cires naturelles animales, on peut citer la cire d'abeille, la lanoline et ses dérivés

Parmi les cires naturelles végétales, on peut citer les cires Candellila, d'Ourrury, de Carnauba, du Japon, le beurre de cacao, les cires de fibres de lièges ou de canne sucre ; les huiles hydrogénées concrètes 25° C telles que l'huile de jojoba hydrogénée l'huile de ricin hydrogénée, l'huile de palme hydrogénée, l'huile de coco hydrogénée ; les esters gras et les glycérides.

Parmi les cires synthétiques, on peut citer les cires de silicone, les cires obtenues par synthèse de Fischer-Tropsch, les cires de polyéthylène, les cires de polytétrafluoroéthylène (PTFE) ou leurs mélanges.On peut citer également les mélanges de cires décrites dans le brevet CLARIANT WO003104330

Les particules de cire micronisées selon l'invention peuvent être constituées d'un mélange d'au moins une cire naturelle et d'au moins une cire synthétique. Les particules de cire micronisées selon l'invention peuvent être également constituées d'un mélange de plusieurs cires naturelles ou d'un mélange de plusieurs cires synthétiques.

Parmi les particules de cire naturelle micronisées utilisables selon l'invention, on peut citer les particules de cire de Carnauba de point de fusion 83-86°C, de densité 0,99 g/cm³ et de taille moyenne 6-8 µm comme le produit commercial vendu sous le nom commercial ®MICROCARE 350 par la société MICRO POWDERS.

Parmi les particules micronisées de cire constituées d'un mélange de cire naturelle et de cire synthétique utilisables selon l'invention, on peut citer les particules constituées de cire de Carnauba et de cire de polyéthylène ayant de point de fusion allant de 107 à 114 °C , une densité de 0,96-0,97 g/cm³ et une taille moyenne de particule allant de 4 à 6 µm comme les produits commerciaux vendus sous la dénomination commerciale ®MICROCARE 300 et 310 par la société MICRO POWDERS.

Parmi les particules micronisées à base de cire synthétique utilisables selon l'invention , on peut citer
- les particules de polytétrafluoroéthylène qui ont un point de fusion supérieur à 316°C, une densité d'environ 2,2 et une taille moyenne de particule allant de 5 à 13 µm comme les produits vendus sous le nom commercial MICROSLIP 519 et MICROSLIP 519 L par la société MICRO POWDERS
- les particules constituées d'un mélange de cire de polyéthylène et de cire de polytétrafluoroéthylène qui ont un point de fusion allant de 115 à 125°C, une densité de 0,99 à 1,1 g/cm³ et une taille moyenne de particule allant de 9 à 12 tels que les produits vendus sous le nom commercial ®MICROSILK 418 et ®MICROSILK 419 par la société MICRO POWDERS.
- les particules de polyéthylène qui ont un point de fusion supérieur à 100 °C, une densité de 0,96 à 0,98 g/cm³ et une taille moyenne de particule allant de 5 à 40 µm comme les produits vendus sous le nom commercial ®MICROTHENE FN-500 et ®MICROTHENE FN-510 par la société Equistar Chemical LP ou les produits vendus sous le nom commercial ®ACUMIST A-6 et ®ACUMIST B-18 par la société Allied Signal Corp

Selon une forme particulièrement préférée de l'invention, on utilisera des particules de polyéthylène de taille moyenne allant de 1 à 12 µm, un point de fusion allant 105 à 135°C et une densité allant de 0,94 à 0,97 g/cm³ en particulier les poudres de polyéthylène vendues sous les noms commerciaux ®MICROPOLY 200, 210, 220, 220L et 250 S par la société MICRO POWDERS.

Les particules de cire selon l'invention sont de préférence présentes dans des quantités allant de 0,5 à 15% en poids et plus préférentiellement de 1 à 10% en poids par rapport au poids total de la composition.

Les compositions selon l'invention peuvent contenir en plus d'autres actifs déodorants.

Au sens de la présente invention, on entend par "actif déodorant" toute substance capable de réduire ou supprimer le flux sudoral et/ou d'absorber la sueur humaine et/ou de masquer, absorber, améliorer ou réduire l'odeur désagréable résultant de la décomposition de la sueur humaine par des bactéries.

Les actifs déodorants additionnels peuvent être des agents bactériostatiques ou des agents bactéricides le 2,4,4'-trichloro-2'-hydroxydiphényléther (®Triclosan), le 2,4-dichloro-2'-hydroxydiphényléther, le 3',4',5'-trichlorosalicylanilide, la 1-(3',4'-dichlorophenyl)-3-(4'-chlorophenyl)urée (®Triclocarban) ou le 3,7,11-triméthyldodéca-2,5,10-triénol (®Farnesol) ; les sels d'ammonium quaternaires comme les sels de cetyltrimethylammonium, les sels de cétylpyridinium

Parmi les actifs déodorants additionnels, on peut aussi citer également les sels de zinc comme le salicylate de zinc, le sulfate de zinc, le chlorure de zinc, le lactate de zinc, le phénolsulfonate de zinc ; la chlorhexidine et les sels; le bicarbonate de sodium ; le monocaprate de diglycérol, le monolaurate de diglycérol , monolaurate de glycérol ; les sels de polyhexaméthylène biguanide.

Les actifs déodorants additionnels peuvent être présents dans la composition selon l'invention à raison d'environ 0,001 à 40% en poids par rapport à la composition totale, et de préférence à raison d'environ 0,1 à 25% en poids.

La composition cosmétique anti-transpirante selon l'invention peut se présenter sous forme plus ou moins épaissie distribuée en tube ou en grille ; sous forme de roll-on conditionnée sous forme de bille et contenir à cet égard les ingrédients généralement utilisés dans ce type de produits et bien connus de l'homme de l'art.

La composition cosmétique anti-transpirante selon l'invention qui est une émulsion huile-dans-eau contient en général au moins un émulsionnant.

Comme émulsionnants, on peut citer par exemple les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

On peut citer également les mélanges émulsionnants alcool gras/alkylpolyglycoside tels que sont décrits dans les demandes WO92/06778, WO95/13863 et WO98/47610 comme les produits commerciaux vendus par la société SEPPIC sous les appellation MONTANOV ®.

La phase grasse conforme aux émulsions huile-dans-eau selon l'invention comprend un ou plusieurs composés hydrophobes qui rendent ladite phase non-miscible dans l'eau.

La phase grasse contient de préférence une ou plusieurs huiles émollientes siliconée ou hydrocarbonée, volatiles ou non-volatiles. Ces huiles émollientes sont notamment décrites dans les brevets US 4,822,596 et US 4,904,463.

Les huiles émollientes selon l'invention peuvent être choisies parmi des produits du type silicone volatile, des silicones non-volatiles et d'autres émollients non-volatils.

Les silicones volatiles sont définies de façon connue comme des composés volatils à température ambiante. (20-25°C). Elles présentent en général une vapeur de pression à 25°C allant de 1 ou 10 Pa à 2kPa.

On peut citer parmi ces composés les silicones volatiles cycliques et linéaires du type diméthylsiloxane dont les chaines comprennent de 3 à 9 résidus siliconés.

De préférence on choisit les cyclométhicones tétramère (D4), pentamère (D5) ou hexamère (D6).

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de «®Dow Corning 245 Fluid» ; les polyalkylarylsiloxanes comme par exemple les polyméthylphénylsiloxanes, commercialisés par la société Dow Corning sous le nom de «®Dow Corning 556 Fluid» ; les copolymères polyéther et siloxane, comme par exemple les diméthicones copolyols.

Parmi les huiles émollientes hydrocarbonées non-volatiles utilisables dans la présente invention, on peut citer par exemple : les dérivés hydrocarbonés liquides comme les huiles minérales et les polyisobutène hydrogénés et plus particulièrement les polydécenes, les paraffines et isoparaffines ayant au moins 10 atomes de carbone , les alcools gras liquides à température ambiante tels que l'alcool isostéarique ou l'octyl dodécanol ; les esters aliphatiques d'alcools en C₃-C₁₈ avec des acides en C₃-C₁₈ comme isopropyl myristate, lauryl myristate, isopropyl palmitate , diisopropyl sebacate, diisopropyl adipate ; les esters aromatiques de l'acide benzoïque avec des alcools en C₁₂-C₁₈ et leurs mélanges comme les alkylbenzoates en C₈-C₁₈ les éthers d'alcools gras aliphatiques tels que les éthers d'alcool myristique comme le PPG-3 myristyl éther et les alkyl(C₁-C₄)éthers de polyglycols comme le PPG-4 butyl éther.

Afin d'améliorer l'homogénéité du produit, on peut utiliser des agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom ®TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

Les compositions selon l'invention peuvent également contenir en plus au moins une poudre organique.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ®ORGASOL par la société Atochem ; les microsphères à base de copolymères acryliques, telles que celles en copolymère diméthacrylate d'éthylène glycol/ methacrylate de lauryle vendues par la société Dow Corning sous la dénomination de ®POLYTRAP ; les microsphères de polyméthacrylate de méthyle, commercialisées sous la dénomination ®MICROSPHERE M-100 par la société Matsumoto ou sous la dénomination ®COVABEAD LH85 par la société Wackherr; les poudres de copolymère éthylène-acrylate, comme celles commercialisées sous la dénomination ®FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination ®EXPANCEL par la société Kemanord Plast sous les références ®551 DE 12 (granulométrie d'environ 12 µm et masse volumique 40 kg/m3), ®551 DE 20 (granulométrie d'environ 30 µm et masse volumique 65 kg/m3), ®551 DE 50 (granulométrie d'environ 40 µm), ou les microsphères commercialisées sous la dénomination ®MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination ®TOSPEARL par la société Toshiba Silicone, notamment TOSPEARL 240 ; les poudres d'aminoacides telles que la poudre de Lauroyllysine commercialisée sous la dénomination ®AMIHOPE LL-11 par la Société Ajinomoto.

La phase aqueuse desdites émulsions contient de l'eau et en général d'autres solvants solubles ou miscibles dans l'eau. Les solvants solubles ou miscibles dans l'eau comprennent les mono alcools à chaîne courte par exemple en C₁-C₄ comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine.

La composition cosmétique selon l'invention peut comprendre en outre des adjuvants cosmétiques choisis les adoucissants, les antioxydants, les opacifiants, les stabilisants, les agents hydratants, les vitamines, les parfums, des bactéricides, les conservateurs, les polymères, les parfums, les agents épaississants, ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la ®BENTONE GEL MIO vendue par la société NL INDUSTRIES ou la ®VEEGUM ULTRA, vendue par la société POLYPLASTIC.

Les quantités de ces différents constituants pouvant être présents dans la composition cosmétique selon l'invention sont celles classiquement utilisées dans les crèmes, sticks-grilles et roll-ons anti-transpirants.

Les compositions selon l'invention peuvent également contenir également un ou plusieurs autres agents structurants ou gélifiants de la phase huileuse tels que ceux évoqués précédemment dans la description tels que les alcools gras solides linéaires et/ou les cires ; les acides gras ou leurs sels (acide stérique, stéarate de sodium, acide 12-hydroxystéarique) ; les dibenzylidene alditols (DBS) ; le lanostérol, les dérivés du N-acyl amino-acide ; les dérivés de di ou triacides carboxyliques comme les alkyl N, N'-dialkylsuccinamides (ie : dodécyl N, N'-dibutylsuccinamide) ;les organopolysiloxane élastomères tels que ceux décrits dans la demande WO97/44010.

La composition selon l'invention peut encore être pressurisée et être conditionnée dans un aérosol; elle contient alors les propulseurs généralement utilisés dans ce type de produits et bien connus de l'homme de l'art, comme par exemple le diméthyléther ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon@ et Dymel®, et en particulier le monofluorotrichlorométhane, le difluorodichlorométhane, le tétrafluorodichloroéthane et le 1,1-difluoroéthane vendu notamment sous la dénomination commerciale ®DYMEL 152 A par la société DUPONT.

On peut également utiliser en tant qu'agent propulseur le gaz carbonique, le protoxyde d'azote, l'azote ou l'air comprimé.

L'agent propulseur représente alors de préférence de 20 à 85% en poids du poids

La présente invention a également pour objet un procédé cosmétique pour traiter la transpiration humaine, consistant à appliquer sur la surface axillaire une quantité efficace d'une composition telle que définie ci-dessus.

La présente invention a pour objet également un procédé pour traiter les odeurs corporelles humaines, consistant à appliquer sur la surface axillaire une quantité efficace d'une composition telle que définie précédemment.

La présente invention a pour objet également l'utilisation de particules de cire ayant une taille moyenne inférieure à 50µm et un point de fusion supérieur à 80 °C dans une composition anti-transpirante du type émulsion huile-dans-eau dans le but de réduire le temps de séchage et l'effet collant de la composition après application sur la peau.

Des exemples concrets, mais nullement limitatif, illustrant l'invention vont maintenant être donné.

On réalise les deux roll-on anti-transpirants de composition suivante :

| **Phase** | **Ingrédients** | **Ex 1** | **(*)Ex 2** |
|---|---|---|---|
| A | PPG-15 stearyl ether (®ARLAMOL E) | 3,00 | 3,00 |
| | Diméthicone 200cst (®DOW CORNING 200 FLUID) | 0,50g | 0,50g |
| | Ceteareth-33 (®SIMULSOL CS) | 1,25 | 1,25 |
| | Alcool cétéarylique | 2,50 | 2,50 |
| C | Particules de cire de polyéthylène de taille moyenne 8-10 µm, de point de fusion e 123-125°C et de densité 0,96 (®MICROPOLY 220 L de Micro Powders) | 5 | - |
| B | Hydroxychlorure d'aluminium en dispersion aqueuse à 50% (®CHORHYDROL 50% USP) | 30,0g | 30,0g |
| | Eau qsp | 100 g | 100g |
| D | Parfum | 1,0 | 1,00 |
| | Conservateur | qs | qs |

| | | | |
|---|---|---|---|
| (*) : hors invention | | | |

Les roll-ons 1 et 2 sont préparés selon le mode opératoire suivant :
Les phases A et B sont portées chacune à 80°C.
L'émulsion est réalisée sous agitation en versant A dans B.
Refroidissement vers 40°C et introduction sous agitation de D puis C

On effectue ensuite un test comparatif sur le temps de séchage et sur l'effet collant de chaque roll-on après application selon le protocole suivant :

On utilise un analyseur de texture présentant les caractéristiques suivantes :
**Appareil** ®TEXTURE EXPERT TA-XT2 par RHEO
**Préréglages**

| | |
|---|---|
| Mobile : | Cylindre de 10 mm en ébonite |
| Pré-vitesse de descente du mobile | 2 mm/s |
| Vitesse de descente du mobile | 10 mm/s |
| Vitesse de remontée du mobile | 5 mm/s |
| Force exercée sur la plaque de verre : | 40g |
| Déplacement : | 0,5 mm |
| Temps | 1,5 s |
| Force de déclenchement | 20 g |
| Vitesse d'acquisition | 200 pps |

On applique chaque produit anti-transpirant N°1 et N°2 avec un tire film sur une plaque de verre. On mesure la force maximale d'adhérence exercée par le mobile qui correspond à l'intensité du collant toutes les minutes et on effectue un nettoyage du mobile après chaque mesure.

On effectue cette opération jusqu'à ce la force maximale soit nulle à savoir jusqu'au terme du séchage. Le temps de séchage est déterminé à ce moment précis.

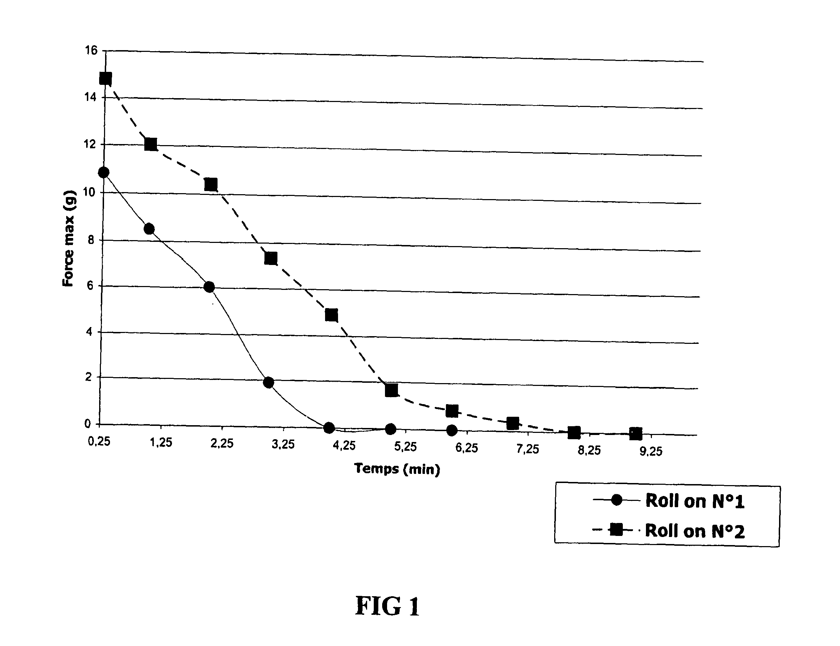

Les résultats des tests sont représentés à la figure 1 par la courbe de variation de l'intensité du collant en fonction du temps.

On constate que le roll-on anti-transpirant N°1 selon l'invention contenant des particules de polyéthylène micronisées sèche beaucoup plus rapidement et présente un effet collant sensiblement plus faible que le roll-on anti-transpirant N°2 sans charge.

## Revendications

1. Composition cosmétique du type émulsion huile-dans-eau, **caractérisée par le fait qu'**elle contient :
(A) au moins un actif anti-transpirant ;
(B) au moins des particules de cire ayant une taille moyenne inférieure à 50µm et un point de fusion supérieur à 80°C.

2. Composition selon la revendication 1, où l'actif anti-transpirant est choisi parmi les sels d'aluminium et/ou de zirconium ; les complexes d'hydroxychlorure de zirconium et d'hydroxychlorure d'aluminium avec un acide aminé

3. Composition selon la revendication 2, où l'actif anti-transpirant est choisi parmi le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

4. Composition selon la revendication 2, où l'actif anti-transpirant est choisi parmi l'aluminium zirconium octachlorohydrate, l'aluminium zirconium pentachlorohydrate, l'aluminium zirconium tetrachlorohydrate, l'aluminium zirconium trichlorohydrate.

5. Composition selon la revendication 2, où l'actif anti-transpirant est choisi parmi les complexes d'hydroxychlorure de zirconium et d'hydroxychlorure d'aluminium avec la glycine (ZAG).

6. Composition selon la revendication 5, où l'actif anti-transpirant est choisi parmi les complexes aluminium zirconium octachlorohydrex glycine, aluminium zirconium pentachlorohydrex glycine, aluminium zirconium tetrachlorohydrex glycine et aluminium zirconium trichlorohydrex glycine.

7. Composition selon la revendication 2 ou 3, où l'actif anti-transpirant est le chlorhydrate d'aluminium sous forme activée ou non activée.

8. Composition selon l'une quelconque des revendications 1 à 7, où le ou les actifs anti-transpirants sont présents à raison d'environ 0,001 à 40% en poids par rapport à la composition totale, et de préférence à raison d'environ 0,1 à 25% en poids.

9. Composition selon l'une quelconque des revendications 1 à 7, où les particules de cire ont de préférence une taille moyenne de particules inférieure à 20 µm, un point de fusion supérieur à 100°C et une densité à 25°C allant de 0,9 à 2,5 g/cm³.

10. Composition selon l'une quelconque des revendications 1 à 9, où les particules de cire sont constituées de cire choisie parmi les cires naturelles d'origine minérale, fossile, animale ou végétale; les cires synthétiques ou de leurs mélanges.

11. Composition selon la revendication 10, où les cires naturelles sont choisies parmi les cires microcristallines, la paraffine, le petrolatum, les paraffines, l'ozokérite, la cire de Montan , la cire d'abeille, la lanoline et ses dérivés, la cire de Candellila, d'Ourrury, de Carnauba, du Japon, le beurre de cacao, les cires de fibres de lièges ou de canne sucre ; les huiles hydrogénées concrètes 25° C ; les esters gras et les glycérides concrets 25° C ;

12. Composition selon la revendication 10, où les cires de synthèse sont choisies parmi les cires de silicone ; les cires obtenus par, synthèse de Fischer-Tropsch, les cires de polyéthylène, les cires de polytétrafluoroéthylène ( PTFE) ou leurs mélanges.

13. Composition selon l'une quelconque des revendications précédentes, où les particules de cire sont constituées d'un mélange d'au moins une cire naturelle et d'au moins une cire synthétique.

14. Composition selon l'une quelconque des revendications précédentes, où les particules de cire sont constituées d'un mélange de plusieurs cires naturelles ou d'un mélange de plusieurs cires synthétiques.

15. Composition selon l'une quelconque des revendications précédentes, où les particules de cire sont des particules de cire de Carnauba de point de fusion 83-86°C, de densité 0,99 g/cm³ et de taille moyenne 6-8 µm.

16. Composition selon l'une quelconque des revendications précédentes, où les particules de cire sont constituées d'un mélange de cire de Carnauba et de cire de polyéthylène et ont un point de fusion allant de 107 à 114°C, une densité de 0,96-0,97 g/cm³ et une taille moyenne de particule allant de 4 à 6 µm.

17. Composition selon l'une quelconque des revendications précédentes, où les particules de cire sont constituées de polytétrafluoroéthylène et présentent un point de fusion supérieur à 316°C, une densité d'environ 2,2 et une taille moyenne de particule allant de 5 à 13 µm.

18. Composition selon l'une quelconque des revendications précédentes, où les particules de cire sont constituées d'un mélange de cire de polyéthylène et de cire de polytétrafluoroéthylène et ont un point de fusion allant de 115 à 125°C, une densité de 0,99 à 1,1 g/cm³ et une taille moyenne de particule allant de 9 à 12µm.

19. Composition selon l'une quelconque des revendications précédentes, où les particules de cire sont constituées de polyéthylène et ont un point de fusion supérieur à 100 °C, une densité de 0,96 à 0,98 g/cm³ et une taille moyenne de particule allant de 5 à 40 µm.

20. Composition selon la revendication 19, où les particules de polyéthylène ont une taille moyenne allant de 1 à 12 µm, un point de fusion allant 105 à 135°C et une densité allant de 0,94 à 0,97 g/cm³ .

21. Composition selon l'une quelconque des revendications 1 à 20, où les particules de cire sont présentes dans des quantités allant de 0,5 à 15% en poids et plus préférentiellement de 1 à 10% en poids par rapport au poids total de la composition.

22. Composition selon l'une quelconque des revendications 1 à 21, contenant en plus au moins un actif déodorant additionnel.

23. Composition selon la revendications 22, où l'actif déodorant additionnel est choisi parmi les agents bactériostatiques ou des agents bactéricides.

24. Composition selon la revendication 22 ou 23, où l'actif déodorant additionnel est choisi parmi le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Famesol) ; le bicarbonate de sodium ; les sels d'ammonium quatemaires;les sels de zinc ; la chlorhexidine et les sels; le monocaprate de diglycérol, le monolaurate de diglycérol , monolaurate de glycérol ; les sels de polyhexaméthylène biguanide.

25. Composition selon l'une quelconque des revendications 22 à 24, où l'actif déodorant additionnel est présent à raison d'environ 0,001 à 40% en poids par rapport à la composition totale, et de préférence à raison d'environ 0,1 à 25% en poids.

26. Composition selon l'une quelconque des revendications 1 à 25, **caractérisée par le fait que** la phase grasse contient une ou plusieurs huiles émollientes siliconée ou hydrocarbonée, volatiles ou non-volatiles.

27. Composition selon l'une quelconque des revendications 1 à 26, **caractérisée par le fait qu**'elle contient en plus au moins un agent de suspension.

28. Composition selon l'une quelconque des revendications 1 à 27, **caractérisée par le fait qu**'elle contient en plus au moins une poudre organique.

29. Composition selon l'une quelconque des revendications 1 à 28, **caractérisée par le fait qu**'elle contient en plus des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants.

30. Composition selon l'une quelconque des revendications 1 à 29, **caractérisée par le fait qu**'elle contient en plus un ou plusieurs agents structurants ou gélifiants de la phase grasse.

31. Composition selon l'une quelconque des revendications 1 à 30, **caractérisée par le fait qu'**elle est pressurisée et conditionnée dans un aérosol et qu'elle contient un ou plusieurs agents propulseurs.

32. Utilisation cosmétique d'une composition selon l'une quelconque des revendications 1 à 31, pour réduire le flux sudoral et/ou masquer, améliorer ou réduire l'odeur désagréable résultant de la décomposition de la sueur humaine par des bactéries.

33. Procédé cosmétique de traitement de la transpiration , consistant à appliquer sur la surface de la peau une quantité efficace d'une composition selon l'une quelconque des revendications 1 à 31.

34. Procédé cosmétique pour traiter les odeurs corporelles humaines liées à la transpiration, consistant à appliquer sur la surface de la peau une quantité efficace d'une composition selon l'une quelconque des revendications 1 à 31.

35. Utilisation de particules de cire ayant une taille moyenne inférieure à 50µm et un point de fusion supérieur à 80 °C dans une composition anti-transpirante du type émulsion huile-dans-eau dans le but de réduire le temps de séchage et l'effet collant de la composition après application sur la peau.

## Claims

1. Cosmetic composition of the oil-in-water emulsion type, **characterized in that** it comprises:
(A) at least one antiperspirant active principle;
(B) at least wax particles having a mean size of less than 50 µm and a melting point of greater than 80°C.

2. Composition according to Claim 1, where the antiperspirant active principle is chosen from aluminium and/or zirconium salts; or complexes of zirconium chlorohydrate and of aluminium chlorohydrate with an amino acid.

3. Composition according to Claim 2, where the antiperspirant active principle is chosen from aluminium chlorohydrate in the activated or nonactivated form, aluminium chlorohydrex, the aluminium chlorohydrex polyethylene glycol complex, the aluminium chlorohydrex propylene glycol complex, aluminium dichlorohydrate, the aluminium dichlorohydrex polyethylene glycol complex, the aluminium dichlorohydrex propylene glycol complex, aluminium sesquichlorohydrate, the aluminium sesquichlorohydrex polyethylene glycol complex, the aluminium sesquichlorohydrex propylene glycol complex, or aluminium sulphate buffered by sodium aluminium lactate.

4. Composition according to Claim 2, where the antiperspirant active principle is chosen from aluminium zirconium octachlorohydrate, aluminium zirconium pentachlorohydrate, aluminium zirconium tetrachlorohydrate or aluminium zirconium trichlorohydrate.

5. Composition according to Claim 2, where the antiperspirant active principle is chosen from complexes of zirconium chlorohydrate and of aluminium chlorohydrate with glycine (ZAG).

6. Composition according to Claim 5, where the antiperspirant active principle is chosen from aluminium zirconium octachlorohydrex glycine, aluminium zirconium pentachlorohydrex glycine, aluminium zirconium tetrachlorohydrex glycine and aluminium zirconium trichlorohydrex glycine complexes.

7. Composition according to Claim 2 or 3, where the antiperspirant active principle is aluminium chlorohydrate in the activated or nonactivated form.

8. Composition according to any one of Claims 1 to 7, where the antiperspirant active principle or principles are present in a proportion of approximately 0.001 to 40% by weight, with respect to the total composition, and preferably in a proportion of approximately 0.1 to 25% by weight.

9. Composition according to any one of Claims 1 to 7, where the wax particles preferably have a mean particle size of less than 20 µm, a melting point of greater than 100°C and a density at 25°C ranging from 0.9 to 2.5 g/cm³.

10. Composition according to any one of Claims 1 to 9, where the wax particles are composed of wax chosen from natural waxes of mineral, fossil, animal or vegetable origin; synthetic waxes or their mixtures.

11. Composition according to Claim 10, where the natural waxes are chosen from microcrystalline waxes, paraffin wax, petrolatum wax, paraffin waxes, ozokerite, montan wax, beeswax, lanolin and its derivatives, candelilla wax, ouricury wax, carnauba wax, Japan wax, cocoa butter or cork fibre or sugarcane waxes, hydrogenated oils which are solid at 25°C, or fatty esters and glycerides which are solid at 25°C.

12. Composition according to Claim 10, where the synthetic waxes are chosen from silicone waxes, the waxes obtained by Fischer-Tropsch synthesis, polyethylene waxes, polytetrafluoroethylene (PTFE) waxes or their mixtures.

13. Composition according to any one of the preceding claims, where the wax particles are composed of a mixture of at least one natural wax and of at least one synthetic wax.

14. Composition according to any one of the preceding claims, where the wax particles are composed of a mixture of several natural waxes or of a mixture of several synthetic waxes.

15. Composition according to any one of the preceding claims, where the wax particles are carnauba wax particles with a melting point of 83-86°C, a density of 0.99 g/cm³ and a mean size of 6-8 µm.

16. Composition according to any one of the preceding claims, where the wax particles are composed of a mixture of carnauba wax and of polyethylene wax and have a melting point ranging from 107 to 114°C, a density of 0.96-0.97 g/cm³ and a mean particle size ranging from 4 to 6 µm.

17. Composition according to any one of the preceding claims, where the wax particles are composed of polytetrafluoroethylene and exhibit a melting point of greater than 316°C, a density of approximately 2.2 and a mean particle size ranging from 5 to 13 µm.

18. Composition according to any one of the preceding claims, where the wax particles are composed of a mixture of polyethylene wax and of polytetrafluoroethylene wax and have a melting point ranging from 115 to 125°C, a density of 0.99 to 1.1 g/cm³ and a mean particle size ranging from 9 to 12 µm.

19. Composition according to any one of the preceding claims, where the wax particles are composed of polyethylene and have a melting point of greater than 100°C, a density of 0.96 to 0.98 g/cm³ and a mean particle size ranging from 5 to 40 µm.

20. Composition according to Claim 19, where the polyethylene particles have a mean size ranging from 1 to 12 µm, a melting point ranging from 105 to 135°C and a density ranging from 0.94 to 0.97 g/cm³.

21. Composition according to any one of Claims 1 to 20, where the wax particles are present in amounts ranging from 0.5 to 15% by weight and more preferentially from 1 to 10% by weight, with respect to the total weight of the composition.

22. Composition according to any one of Claims 1 to 21, additionally comprising at least one additional deodorant active principle.

23. Composition according to Claim 22, where the additional deodorant active principle is chosen from bacteriostatic agents or bactericidal agents.

24. Composition according to Claim 22 or 23, where the additional deodorant active principle is chosen from 2,4,4'-trichloro-2'-hydroxydiphenyl ether (Triclosan), 2, 4-dichloro-2'-hydroxydiphenyl ether, 3', 4', 5'-trichlorosalicylanilide, 1- (3',4'-dichlorophenyl) -3- (4'-chlorophenyl) urea (Triclocarban) or 3,7,11-trimethyldodeca-2,5,10-trienol (Farnesol); sodium bicarbonate; quaternary ammonium salts; zinc salts; chlorhexidine and its salts; diglycerol monocaprate, diglycerol monolaurate or glycerol monolaurate; or polyhexamethylene biguanide salts.

25. Composition according to any one of Claims 22 to 24, where the additional deodorant active principle is present in a proportion of approximately 0.001 to 40% by weight, with respect to the total composition, and preferably in a proportion of approximately 0.1 to 25% by weight.

26. Composition according to any one of Claims 1 to 25, **characterized in that** the fatty phase comprises one or more volatile or nonvolatile emollient oils of silicone or hydrocarbon nature.

27. Composition according to any one of Claims 1 to 26, **characterized in that** it additionally comprises at least one suspending agent.

28. Composition according to any one of Claims 1 to 27, **characterized in that** it additionally comprises at least one organic powder.

29. Composition according to any one of Claims 1 to 28, **characterized in that** it additionally comprises cosmetic adjuvants chosen from waxes, softeners, antioxidants, opacifiers, stabilizers, moisturizing agents, vitamins, fragrances, bactericides, preservatives, polymers or thickening agents.

30. Composition according to any one of Claims 1 to 29, **characterized in that** it additionally comprises one or more structuring or gelling agents for the fatty phase.

31. Composition according to any one of Claims 1 to 30, **characterized in that** it is pressurized and packaged in an aerosol and **in that** it comprises one or more propellants.

32. Cosmetic use of a composition according to any one of Claims 1 to 31 to reduce the flow of sweat and/or to mask, improve or reduce the unpleasant odour resulting from the decomposition of human sweat by bacteria.

33. Cosmetic process for the treatment of perspiration which consists in applying, to the surface of the skin, an effective amount of a composition according to any one of Claims 1 to 31.

34. Cosmetic process for treating human body odours related to perspiration which consists in applying, to the surface of the skin, an effective amount of a composition according to any one of Claims 1 to 31.

35. Use of wax particles having a mean size of less than 50 µm and a melting point of greater than 80°C in an antiperspirant composition of the oil-in-water emulsion type for the purpose of reducing the drying time and the stickiness effect of the composition after application to the skin.

## Patentansprüche

1. Kosmetische Zusammensetzung vom Typ einer Öl-in-Wasser-Emulsion, **dadurch gekennzeichnet, dass** sie enthält:
(A) zumindest ein Antitranspirant;
(B) zumindest Wachspartikel, die eine mittlere Größe unter 50 µm und einen Schmelzpunkt über 80 °C aufweisen.

2. Zusammensetzung nach Anspruch 1, wobei das Antitranspirant unter Salzen von Aluminium und/oder Zirconium; den Komplexen von Zirconiumhydroxychlorid und Aluminiumhydroxychlorid mit einer Aminosäure ausgewählt ist.

3. Zusammensetzung nach Anspruch 2, wobei das Antitranspirant unter Aluminium Chlorhydrate in aktivierter oder nichtaktivierter Form, Aluminium Chlorohydrex, dem Komplex Aluminium Chlorohydrex Polyethylene Glycol, dem Komplex Aluminium Chlorohydrex Propylene Glycol, Aluminium Dichlorohydrate, dem Komplex Aluminium Dichlorohydrex Polyethylene Glycol, dem Komplex Aluminium Dichlorohydrex Propylene Glycol, Aluminium Sesquichlorohydrate, dem Komplex Aluminium Sesquichlorohydrex Polyethylene Glycol, dem Komplex Aluminium Sesquichlorohydrex Propylene Glycol, mit Natriumlactat und A-luminiumlactat gepuffertem Aluminiumsulfat ausgewählt ist.

4. Zusammensetzung nach Anspruch 2, wobei das Antitranspirant unter Aluminium Zirconium Octachlorohydrate, Aluminium Zirconium Pentachlorohydrate, Aluminium Zirconium Tetrachlorohydrate, Aluminium Zirconium Trichlorohydrate ausgewählt ist.

5. Zusammensetzung nach Anspruch 2, wobei das Antitranspirant unter den Komplexen von Zirconiumhydroxychlorid und Aluminiumhydroxychlorid mit Glycin (ZAG) ausgewählt ist.

6. Zusammensetzung nach Anspruch 5, wobei das Antitranspirant unter den Komplexen Aluminium Zirconium Octachlorohydrex Glycine, Aluminium Zirconium Pentachlorohydrex Glycine, Aluminium Zirconium Tetrachlorohydrex Glycine und Aluminium Zirconium Trichlorohydrex Glycine ausgewählt ist.

7. Zusammensetzung nach Anspruch 2 oder 3, wobei das Anti-transpirant das Aluminiumhydroxychlorid in aktivierter oder nichtaktivierter Form ist.

8. Zusammensetzung nach einem der Ansprüche 1 bis 7, wobei das oder die Antitranspirantien in einer Menge von etwa 0,001 bis 40 Gew.-%, bezogen auf die gesamte Zusammensetzung, und vorzugsweise von etwa 0,1 bis 25 Gew.-% vorliegen.

9. Zusammensetzung nach einem der Ansprüche 1 bis 7, wobei die Wachspartikel vorzugsweise eine mittlere Partikelgröße unter 20 µm, einen Schmelzpunkt über 100 °C und eine Dichte von 0,9 bis 2,5 g/cm³ bei 25 °C haben.

10. Zusammensetzung nach einem der Ansprüche 1 bis 9, wobei die Wachspartikel aus einem Wachs bestehen, das unter den natürlichen Wachsen mineralischer, fossiler, tierischer oder pflanzlicher Herkunft; synthetischen Wachsen oder ihren Gemischen ausgewählt ist.

11. Zusammensetzung nach Anspruch 10, wobei die natürlichen Wachse unter mikrokristallinen Wachsen, Paraffin, Petrolatum, Paraffinen, Ozokerit, Montanwachs, Bienenwachs, Lanolin und seinen Derivaten, Candelillawachs, Ouricurywachs, Carnaubawachs, Japanwachs, Kakaobutter, den Wachsen aus Korkfasern oder aus Zuckerrohr; bei 25 °C festen hydrierten Ölen; Fettsäureestern und Glyceriden, die bei 25 °C fest sind, ausgewählt sind.

12. Zusammensetzung nach Anspruch 10, wobei die synthetischen Wachse unter Siliconwachsen; Wachsen, die durch Fischer-Tropsch-Synthese erhalten werden, Polyethylenwachsen, Polytetrafluorethylenwachsen (PTFE) oder ihren Gemischen ausgewählt sind.

13. Zusammensetzung nach einem der vorhergehenden Ansprüche, wobei die Wachspartikel aus einem Gemisch aus mindestens einem natürlichen Wachs und mindestens einem synthetischen Wachs bestehen.

14. Zusammensetzung nach einem der vorhergehenden Ansprüche, wobei die Wachspartikel aus einem Gemisch mehrerer natürlicher Wachse oder einem Gemisch mehrerer synthetischer Wachse bestehen.

15. Zusammensetzung nach einem der vorhergehenden Ansprüche, wobei die Wachspartikel Partikel von Carnaubawachs mit einem Schmelzpunkt von 83 bis 86°C, einer Dichte von 0,99 g/cm³ und einer mittleren Größe von 6 bis 8 µm sind.

16. Zusammensetzung nach einem der vorhergehenden Ansprüche, wobei die Wachspartikel aus einem Gemisch von Carnaubawachs und Polyethylenwachs bestehen und einen Schmelzpunkt von 107 bis 114 °C, eine Dichte von 0,96 bis 0,97 g/cm³ und eine mittlere Partikelgröße von 4 bis 6 µm aufweisen.

17. Zusammensetzung nach einem der vorhergehenden Ansprüche, wobei die Wachspartikel aus Polytetrafluorethylen bestehen und einen Schmelzpunkt über 316 °C, eine Dichte von etwa 2,2 und eine mittlere Partikelgröße von 5 bis 13 µm aufweisen.

18. Zusammensetzung nach einem der vorhergehenden Ansprüche, wobei die Wachspartikel aus einem Gemisch von Polyethylenwachs und Polytetrafluorethylenwachs bestehen und einen Schmelzpunkt im Bereich von 115 bis 125 °C, eine Dichte von 0,99 bis 1,1 g/cm³ und eine mittlere Partikelgröße von 9 bis 12 µm aufweisen.

19. Zusammensetzung nach einem der vorhergehenden Ansprüche, wobei die Wachspartikel aus Polyethylen bestehen und einen Schmelzpunkt über 100 °C, eine Dichte von 0,96 bis 0,98 g/cm³ und eine mittlere Partikelgröße von 5 bis 40 µm aufweisen.

20. Zusammensetzung nach Anspruch 19, wobei die Polyethylenpartikel eine mittlere Größe im Bereich von 1 bis 12 µm, einen Schmelzpunkt von 105 bis 135 °C und eine Dichte von 0,94 bis 0,97 g/cm³ haben.

21. Zusammensetzung nach einem der Ansprüche 1 bis 20, wobei die Wachspartikel in einer Menge von 0,5 bis 15 Gew.-% und bevorzugt 1 bis 10 Gew.-%, bezogen auf das Gesamtgewicht der Zusammensetzung, enthalten sind.

22. Zusammensetzung nach einem der Ansprüche 1 bis 21, die ferner zumindest einen zusätzlichen desodorierenden Wirkstoff enthält.

23. Zusammensetzung nach Anspruch 22, wobei der zusätzliche desodorierende Wirkstoff unter bakteriostatischen Stoffen oder bakteriziden Stoffen ausgewählt ist.

24. Zusammensetzung nach Anspruch 22 oder 23, wobei das zusätzliche desodorierende Mittel unter 2,4,4'-Trichlor-2'-hydroxydiphenylether (Triclosan), 2,4-Dichlor-2'-hydroxydiphenylether, 3',4',5'-Trichlorsalicylanilid, 1-(3',4'-Dichlorphenyl)-3-(4'-chlorphenyl)harnstoff (Triclocarban) oder 3,7,11-Trimethyldodeca-2,6,10-trienol (Farnesol); Natriumhydrogencarbonat; quartären Ammoniumsalzen; Zinksalzen; Chlorhexidin und seinen Salzen; Diglycerylmonocaprat, Diglycerylmonolaurat, Glycerylmonolaurat; den Salzen von Polyhexamethylenbiguanid ausgewählt ist.

25. Zusammensetzung nach einem der Ansprüche 22 bis 24, wobei der zusätzliche desodorierende Wirkstoff in einer Menge von etwa 0,001 bis 40 Gew.-%, bezogen auf die gesamte Zusammensetzung, und vorzugsweise in einer Menge von etwa 0,1 bis 25 Gew.-% vorliegt.

26. Zusammensetzung nach einem der Ansprüche 1 bis 25, **dadurch gekennzeichnet, dass** die Fettphase ein oder mehrere weichmachende Siliconöle oder Kohlenwasserstofföle enthält, die flüchtig oder nichtflüchtig sind.

27. Zusammensetzung nach einem der Ansprüche 1 bis 26, **dadurch gekennzeichnet, dass** sie darüber hinaus zumindest ein Suspendiermittel enthält.

28. Zusammensetzung nach einem der Ansprüche 1 bis 27, **dadurch gekennzeichnet, dass** sie ferner zumindest ein organisches Pulver aufweist.

29. Zusammensetzung nach einem der Ansprüche 1 bis 28, **dadurch gekennzeichnet, dass** sie darüber hinaus kosmetische Zusatzstoffe enthält, die unter Wachsen, beruhigenden Stoffen, Antioxidantien, Trübungsmitteln, Stabilisatoren, Hydratisierungsmitteln, Vitaminen, Parfums, Bakteriziden, Konservierungsmitteln, Polymeren, Parfums und Verdickungsmitteln ausgewählt sind.

30. Zusammensetzung nach einem der Ansprüche 1 bis 29, **dadurch gekennzeichnet, dass** sie ferner ein oder mehrere strukturgebende Mittel oder Gelbildner für die Fettphase enthält.

31. Zusammensetzung nach einem der Ansprüche 1 bis 30, **dadurch gekennzeichnet, dass** sie unter Druck steht und als Aerosol konfektioniert ist und dass sie ein oder mehrere Treibmittel enthält.

32. Kosmetische Verwendung einer Zusammensetzung nach einem der Ansprüche 1 bis 31, um die Schweißsekretion zu verringern und/oder den unangenehmen Geruch zu überdecken, zu verbessern oder zu mindern, der durch die Zersetzung des menschlichen Schweißes durch Bakterien entsteht.

33. Kosmetisches Verfahren zur Behandlung der Transpiration, das das Auftragen einer wirksamen Menge einer Zusammensetzung nach einem der Ansprüche 1 bis 31 auf die Oberfläche der Haut umfasst.

34. Kosmetisches Verfahren zur Behandlung der menschlichen Körpergerüche, die in Verbindung mit der Transpiration stehen, das das Auftragen einer wirksamen Menge einer Zusammensetzung nach einem der Ansprüche 1 bis 31 auf die Oberfläche der Haut umfasst.

35. Verwendung von Wachspartikeln, die eine mittlere Größe unter 50 µm und einen Schmelzpunkt über 80 °C haben, in einer schweißverhütenden Zusammensetzung vom Typ einer Öl-in-Wasser-Emulsion, um die Trocknungszeit und die klebrige Wirkung der Zusammensetzung nach dem Auftragen auf die Haut zu verringern.
